# EUROPÄISCHE PATENTSCHRIFT

(11) **EP 1 588 168 B1**
(45) Veröffentlichungstag und Bekanntmachung des Hinweises auf die Patenterteilung: **08.07.2009**
(21) Anmeldenummer: 04704574.5
(22) Anmeldetag: 23.01.2004
(51) Int. Cl.: G01N 33/543, G01N 33/555

(54) **VERFAHREN ZUM SEPARIEREN UND NACHWEISEN VON ZUMINDEST EINEM ANALYTEN IN BIOLOGISCHEN MATRIZES**
METHOD FOR SEPARATING AND DETECTING AT LEAST ONE ANALYTE IN BIOLOGICAL MATRICES
PROCEDE POUR SEPARER ET METTRE EN EVIDENCE LA PRESENCE D'AU MOINS UN ANALYTE DANS DES MATRICES BIOLOGIQUES

(30) Priorität: 26.01.2003 DE 10303043; 16.06.2003 DE 10328181
(43) Veröffentlichungstag der Anmeldung: 26.10.2005
(73) Patentinhaber: Eberhard-Karls-Universität Tübingen Universitätsklinikum, 72076 Tübingen (DE); Universität Kaiserlautern, 67663 Kaiserslautern (DE)
(72) Erfinder: NORTHOFF, Hinnak, 72076 Tübingen (DE); GEHRING, Frank, 72364 Obernheim (DE); ZIEGLER, Christiane, 67663 Kaiserslautern (DE)
(74) Vertreter: Otten, Hajo
(86) Internationale Anmeldenummer: PCT/EP2004/000529
(87) Internationale Veröffentlichungsnummer: WO 2004/067130

(56) Entgegenhaltungen:
- EP-A- 0 453 224
- WO-A1-01/02857
- DE-C- 19 826 617
- US-B1- 6 289 717

## Beschreibung

Die vorliegende Erfindung betrifft ein Verfahren zum Separieren von zumindest einem Analyten aus einer komplexen, vorzugsweise biologischen Matrix, ein Verfahren zum Nachweis eines Analyten in einer komplexen, vorzugsweise biologischen Matrix, ein Verfahren zum Nachweis von in einer biologischen Probe, vorzugsweise einer Blutprobe enthaltenen Antikörpern/Rezeptoren gegen Antigene/Liganden, ein Verfahren zum Nachweis von in einer ersten Blutprobe enthaltenen Antikörpern gegen Erythrozyten in einer zweiten Blutprobe, sowie eine Vorrichtung zum Nachweis eines Analyten in einer komplexen, vorzugsweise biologischen Matrix.

Für den Nachweis von Analyten in komplexen Matrizes ist es häufig erforderlich, den Analyten, der ein Antikörper, ein Rezeptor oder ein beliebiges Biomolekül sein kann, von den anderen Komponenten der Matrix zu separieren, bevor der eigentliche Mess- oder Bestimmungsvorgang erfolgen kann.

Für die chemische Analyse in komplexen Matrizes kommen daher häufig analytische Systeme wie Test Kits, Membranen etc. oder analytische Apparaturen in Kombination mit Membranen zum Einsatz,, wobei die Membranen für die Separation des Analyten von den anderen Stoffen der Matrix notwendig sind. Beispiel für gängige chemische Analysemethoden finden sich in der WO 99/20396.

Bei der Blutanalyse z.B. ist die Zentrifugation ein gängiges Verfahren zur Separierung von Blutbestandteilen für deren nachfolgende weitere Untersuchung.

Die WO 01/02857 beschreibt ein Verfahren, bei dem verschiedene Komponenten aus einer Lösung auf einer schwingungsfähigen Oberfläche gebunden und anschließend durch schrittweise Erhöhung der Schwingungsamplitude wieder abgelöst werden.

Für den Nachweis von Analyten, unter denen im Rahmen der vorliegenden Anmeldung allgemein Moleküle, Bakterien, Viren, Zellen, Antikörper, Rezeptoren, Liganden etc. verstanden werden, werden neuerdings Chemo- oder Biosensoren eingesetzt, auf deren Oberfläche der Analyt abgeschieden wird, um dann unterschiedlichste biologische, chemische oder physikalische Eigenschaften wie bspw. Temperatur, Masse, pH-Wert, Bindungskinetik, Absorption, Spannung etc. zu bestimmen. Wichtig ist dabei, dass die Anlagerung spezifisch für den Analyten ist und unspezifische Anlagerung - wenn überhaupt - nur unterhalb der Nachweisgrenze erfolgt. Hierzu wird im Allgemeinen die Oberfläche des Sensors mit einer Beschichtung versehen, die ein spezifisches Erkennen des Analyten ermöglicht.

Beim Nachweis von Analyten in komplexen, insbesondere biologischen Matrizes wie bspw. Körperflüssigkeiten ist die unspezifische Anlagerung jedoch oft so groß, dass ein Nachweis der spezifischen Anlagerung entweder überhaupt nicht oder nur schwer möglich ist.

Bei Sensoren auf der Basis von Schwingquarzen wird der Nachweis neben der unspezifischen Anlagerung auch noch durch die Masse des Analyten beeinflusst. Eine Änderung in der Schwingungsamplitude oder -frequenz lässt sich nur detektieren, wenn die Masseänderung durch die Belegung mit dem Analyten eine bestimmte Größe erreicht. Haben bspw. die Analyten nur eine geringe Masse, die sich unspezifisch ablagernden Komponenten der Matrix jedoch eine viel größere Masse, so führt schon eine geringe unspezifische Massebelegung zu nicht mehr aussagekräftigen Messsignalen.

Vor diesem Hintergrund besteht Bedarf an einem Verfahren, das es erlaubt, Moleküle, also Analyten, aus komplexen Matrizes nachzuweisen, die wegen störender unspezifischer Ablagerung von anderen Komponenten aus der Matrix und/oder wegen der nur geringen Massezunahme an dem Sensor bei Ablagerung des Analyten bisher nur mit Filterung der Matrix über bspw. Membranen oder andere aufwendige Verfahren nachzuweisen waren.

Das neue Verfahren soll bspw. den Nachweis von Antikörpern in Serum oder Blutplasma sowie eine komplette Blutgruppenbestimmung ermöglichen, also bspw. auch den sogenannten Antikörpersuchtest mit umfassen, aber auch den Nachweis von Viren, Bakterien und anderen Mikropartikeln nicht nur in Körperflüssigkeiten sondern auch in wässrigen Proben bspw. aus der Umweltanalytik ermöglichen.

Es ist daher Aufgabe der vorliegenden Erfindung, ein Verfahren bereitzustellen, mit welchem schnell und ohne großen Aufwand" Analyten aus komplexen Matrizes separiert und/oder nachgewiesen werden können.

Erfingdungsgemäß wird diese Aufgabe gelöst durch ein Verfahren zum Separieren von zumindest einem Analyten aus einer komplexen, vorzugsweise biologischen Matrix unter Verwendung zumindest einer ersten schwingungsfähigen Oberfläche, mit den Schritten:
a) an der ersten Oberfläche werden Trägerpartikel immobilisiert, die zumindest eine Bindungsstelle für den Analyten aufweisen;
b) die Matrix wird mit der ersten Oberfläche in Kontakt gebracht, so dass ggf. in der Matrix enthaltener Analyt an die Trägerpartikel bindet;
c) unspezifisch gebundene Bestandteile der Matrix werden von der ersten Oberfläche entfernt, vorzugsweise abgewaschen oder abgespült;
d) die erste Oberfläche wird in Schwingung versetzt und ihre Schwingungsamplitude erhöht, bis sich die Trägerpartikel von der Oberfläche ablösen; und
e) die Trägerpartikel mit ggf. daran gebundenem Analyten werden in einem Trägermedium aufgenommen.

Die Erfindung wird ferner gelöst durch ein Verfahren zum Nachweis eines Analyten in einer komplexen, vorzugsweise biologischen Matrix, mit den Schritten:
a) der Analyt wird in dem neuen Verfahren von der Matrix separiert, und
b) es wird überprüft, ob an die von der ersten Oberfläche abgelösten Trägerpartikel ein Analyt gebunden hat.

Die Erfindung betrifft vor diesem Hintergrund auch ein Verfahren zum Nachweis von in einer biologischen Probe, vorzugsweise einer Blutprobe enthaltenen Antikörpern/Rezeptoren gegen Antigene/Liganden, unter Verwendung einer ersten schwingungsfähigen Oberfläche, an der Fängermoleküle für Trägerpartikel gebunden sind, auf denen sich die Antigene/Liganden befinden, mit den Schritten:
a) die Trägerpartikel werden mit der ersten Oberfläche in Kontakt gebracht, so dass sie an den Fängermolekülen der ersten Oberfläche immobilisiert werden;
b) die Probe wird mit der Oberfläche in Kontakt gebracht, so dass die Antikörper/Rezeptoren an die Antigene/Liganden auf den Trägerpartikeln binden;
c) unspezifisch gebundene Bestandteile der Probe werden von der Oberfläche entfernt, vorzugsweise abgewaschen oder abgespült;
d) die Oberfläche wird in Schwingung versetzt und ihre Schwingungsamplitude erhöht, bis sich die Trägerpartikel von den Fängermolekülen ablösen;
e) die Trägerpartikel werden mit einer zweiten schwingungsfähigen Oberfläche in Kontakt gebracht, an der Fängermoleküle für die Antikörper/Rezeptoren gebunden sind, wobei vorzugsweise die zweite schwingungsfähige Oberfläche die erste schwingungsfähige Oberfläche ist oder der ersten schwingungsfähigen Oberfläche entspricht bzw. eine aktivierte Goldoberfläche ist; und
h) die zweite schwingungsfähige Oberfläche wird in Schwingung versetzt und ihre Massenbelegung ermittelt.

Ferner betrifft die Erfindung ein Verfahren zum Nachweis von in einer ersten Blutprobe enthaltenen Antikörpern gegen Erythrozyten in einer zweiten Blutprobe, unter Verwendung einer ersten schwingungsfähigen Oberfläche, an der Fängermoleküle für die Erythrozyten der zweiten Blutprobe gebunden sind, mit den Schritten:
a) die zweite Blutprobe wird mit der Oberfläche in Kontakt gebracht, so dass die Erythrozyten an der Oberfläche immobilisiert werden;
b) unspezifisch gebundene Bestandteile der zweiten Blutprobe werden von der Oberfläche entfernt, vorzugsweise abgewaschen;
c) die erste Blutprobe wird mit der Oberfläche in Kontakt gebracht, so dass die Antikörper an die Erythrozyten binden;
d) unspezifisch gebundene Bestandteile der ersten Blutprobe werden von der Oberfläche entfernt, vorzugsweise abgewaschen oder abgespült;
e) die Oberfläche wird in Schwingung versetzt und ihre Schwingungsamplitude erhöht, bis sich die Erythrozyten von den Fängermolekülen ablösen;
f) die Erythrozyten mit ggf. daran gebundenen Antikörpern werden in einem Trägermedium aufgenommen;
g) das Trägermedium wird mit einer zweiten schwingungsfähigen Oberfläche in Kontakt gebracht, an der Fängermoleküle für die Antikörper gebunden sind, wobei vorzugsweise die zweite schwingungsfähige Oberfläche die erste schwingungsfähige Oberfläche ist oder der ersten schwingungsfähigen Oberfläche entspricht bzw. eine aktivierte Goldoberfläche ist; und
h) die zweite schwingungsfähige Oberfläche wird in Schwingung versetzt und ihre Massenbelegung ermittelt.

Schließlich betrifft die Erfindung auch eine Vorrichtung zum Nachweis eines Analyten in einer komplexen, vorzugsweise biologischen Matrix, mit
einer ersten Messzelle, in der ein Analyt aus der Matrix spezifisch an auf einer schwingungsfähigen ersten Oberfläche immobilisierte Trägerpartikel gebunden wird, und Trägerpartikel mit ggf. daran gebundenem Analyten von der Matrix separiert werden;
einer zweiten Messzelle, in der messtechnisch bestimmt wird, ob die Trägerpartikel Analyt gebunden haben; und
einer Einrichtung, um die Trägerpartikel aus der ersten in die zweite Messzelle zu überführen,
wobei die neue Vorrichtung vorzugsweise dazu eingerichtet ist, das erfindungsgemäße Verfahren in seinen verschiedenen Ausgestaltungen durchzuführen.

Die der Erfindung zugrundeliegende Aufgabe wird dadurch vollkommen gelöst.

Im Rahmen der vorliegenden Erfindung wird unter "Blutprobe" nicht nur eine Probe aus Vollblut, sondern auch eine Plasma- oder Serumprobe verstanden.

Unter "Trägerpartikel" wird im Rahmen der vorliegenden Erfindung auch ein "Trägermolekül" verstanden.

Die Erfinder haben erkannt, dass es durch das erfindungsgemäße Verfahren möglich ist, aus einer komplexen Matrix Analyt, wie bspw. Antikörper/Rezeptoren, mit geringer Masse an Trägerpartikel größerer Masse zu binden und über eine schwingungsfähige Oberfläche von den anderen, sich ggf. unspezifisch ablagernden Komponenten der Matrix zu separieren, und anschließend zu prüfen, ob die Trägerpartikel Analyt gebunden haben. Dies kann mit verschiedenen Chemo- und Biosensoren erfolgen, es ist jedoch bevorzugt, wenn schwingungsfähige Oberflächen auch für diesen weiteren Schritt eingesetzt werden. Die Analyten erhalten nämlich während der Separierung bereits eine größere Masse, so dass sie bei Ablagerung auf einer schwingungsfähigen Oberfläche zu einer gut erfassbaren Masseveränderung führen.

Durch das erfindungsgemäße Separierverfahren werden bekannte Chemo- und Biosensoren besser einsetzbar, denn die im Stand der Technik erforderlichen Laborarbeiten zur Separierung der Analyten werden durch die Erfindung erheblich verringert bzw. durch ein automatisierbares Verfahren ersetzt. Bei der kompletten Blutgruppenbestimmung, also bspw. einschließlich Antikörpersuchtest, sowie dem Nachweis von Viren, Bakterien und anderen Mikroorganismen aus Blut kann auf die bisher üblichen serologischen Methoden verzichtet werden. Gleiches gilt für bisher aufwendige Verfahren in der Umweltanalytik, bspw. dem Nachweis von Viren in Gewässern.

Bei dem neuen Verfahren ist es folglich bevorzugt, wenn die schwingungsfähige erste und/oder zweite Oberfläche an einem Schwingquarz, einem Dickenscherschwinger, einer Quarzmikrowaage oder einem Balkenschwinger ausgebildet ist und vorzugsweise mit einer Elektrode, vorzugsweise einer Goldelektrode, weiter vorzugsweise einer aktivierten Goldelektrode versehen ist.

Die Aktivierung von Goldoberflächen kann beispielsweise dadurch erfolgen, dass die Goldoberfläche mit einer Piranha genannten Lösung aus einem Drittel 35%igem H₂0₂ und zwei Drittel 98%igem H₂SO₄ gewaschen wird, wird die Goldelektrode beispielsweise für eine Minute in eine derartige Piranha-Lösung eingelegt, so wird an ihr eine hydrophile Oberfläche geschaffen.

An einer derart aktivierten, reinen Goldoberfläche können Trägermoleküle wie beispielsweise Erythrozyten nur unspezifisch und in sehr geringem Maße binden. Mit Antikörpern beladene Erythrozyten binden dagegen sehr viel stärker an derart aktivierte Goldoberflächen als unbeladene Erythrozyten, da die Antikörper über Disulfidbrücken an das Gold binden.

Die schwingungsfähige erste und/oder zweite Oberfläche bzw. die Elektrode ist dabei vorzugsweise funktionalisiert, weiter vorzugsweise mit einer Beschichtung aus organischem Material versehen ist, sie trägt insbesondere eine Protein A, Antikörper- oder Lektin-Schicht.

Es ist dabei bevorzugt, wenn an der Beschichtung Fängermoleküle, insbesondere Antikörper, Lektine, Biotin oder Avidin gebunden sind, die eine Bindungsstelle für die Trägerpartikel aufweisen, wobei es weiter von Vorteil ist, wenn die Trägerpartikel über darauf befindliche Liganden oder Antigene an der ersten Oberfläche immobilisiert sind.

Das erfindungsgemäße Verfahren kann dabei sowohl mit zwei verschiedenen als auch mit einer einzigen Oberfläche durchgeführt werden, wobei es bei zwei verschiedenen Oberflächen bevorzugt sein kann, wenn die beiden Oberflächen identisch ausgebildet sind, die zweite schwingungsfähige Oberfläche also der ersten schwingungsfähigen Oberfläche entspricht. Beide Oberflächen können mit den gleichen Fängermolekülen beschichtete Oberflächen sein. Die zweite Oberfläche kann auch eine aktivierte Goldoberfläche sein.

Wenn die zweite Oberfläche eine aktivierte Goldoberfläche ist, so binden zunächst die noch unbeladenen Erythrozyten an die erste Oberfläche und werden dann ggf. mit Antikörpern beladen. Die Erythrozyten werden dann von der ersten Oberfläche "abgesprengt" und danach an die zweite schwingungsfähige Oberfläche gebunden.

Wenn die Erythrozyten jetzt mit Antikörpern beladen sind, also in der ersten Blutprobe Antikörper gegen die Erythrozyten aus der zweiten Blutprobe enthalten waren, binden die so beladenen Erythrozyten jetzt an die aktivierte Goldoberfläche. Diese Bindung ermöglicht eine Aussage darüber, ob in der ersten Blutprobe Antikörper gegen Erythrozyten aus der zweiten Blutprobe enthalten waren.

Gleichfalls ist es möglich, bei beschichteten oder mit Fängermolekülen versehenen Oberflächen als zweite Oberfläche die erste Oberfläche oder eine zu der ersten Oberfläche identische, schwingungsfähige Oberfläche zu verwenden. Mit Antikörpern beladene Erythrozyten binden dann an die zweite Oberfläche deutlich schwächer als unbeladene Erythrozyten. Wenn der Test positiv ist, ergibt sich hier eine geringe Masseänderung während sich im obigen Fall, wo die zweite Oberfläche eine aktivierte Goldfläche ist, eine größere Massebelegung ergibt.

Vorzugsweise sind die Trägerpartikel ausgewählt aus der Gruppe: Latexpartikel, biologische Makromoleküle, biologische Zellen, Mikroorganismen, Bakterien, Blutzellen, insbesondere Erythrozyten, Partikel aus Zellulose, Polysaccharide, abgetötete Mikroorganismen und Zellen, magnetische Beads, wobei insbesondere jedes Trägerpartikel eine größere, vorzugsweise eine erheblich größere Masse hat als der Analyt.

Auf diese Weise erhalten die Analyten während des Separierens sozusagen eine größere Masse, so dass sie mit einem Masseschwinger leichter und vor allem genauer nachgewiesen werden können.

Es ist weiter bevorzugt, wenn der Analyt ausgewählt ist aus der Gruppe: Biomoleküle, insbesondere Antigene oder Antikörper, vorzugsweise Antikörper gegen eine Blutgruppe, gegen Viren, gegen Mikroorganismen oder sonstige Mikropartikel, und wenn die Matrix ausgewählt ist aus der Gruppe: Körperflüssigkeiten, insbesondere Vollblut, Blutserum, Blutplasma, Urin, Speichel, Tränenflüssigkeit, Liquor cerebrospinalis, Lymphe, Sputum; Zellextrakte; Zellkulturüberstände; wässrige Proben.

Damit lassen sich "leichte" Analyten aus verschiedensten Bereichen separieren und nachweisen.

Ferner ist es bevorzugt, wenn ein gebundener Analyt zu einer messtechnisch erfassbaren Veränderung der biologischen, chemischen oder physikalischen Eigenschaften der Trägerpartikel führt.

Zusätzlich zu oder anstatt einer Erhöhung der Masse der Analyten kann die Separierung auch dazu führen, dass die Trägermoleküle durch den gebundene Analyten eine mit Chemo- oder Biosensoren messtechnisch erfassbare Veränderung erfahren. Dies kann bspw. die Bindungsmöglichkeit an Antikörper sein, die nicht unmittelbar an die Trägerpartikel, wohl aber an den Analyten binden, so dass nur solche Trägerpartikel an die Sensoren gebundene werden, die zuvor einen Analyten gebunden haben.

Allgemein ist es dabei bevorzugt, wenn die Trägerpartikel über daran gebundenen Analyten an einen Chemo- oder Biosensor gebunden werden, wobei der Chemo- oder Biosensor vorzugsweise als Schwingquarz, Dickenscherschwinger, Quarzmikrowaage oder Balkenschwinger mit der schwingungsfähigen zweiten Oberfläche ausgebildet ist.

Bei der neuen Vorrichtung ist es bevorzugt, wenn die Einrichtung Ventile und Schläuche umfasst, über die die einzelnen Flüssigkeiten zu und von den Messzellen geleitet werden.

Weitere Vorteile ergeben sich aus den nachstehenden Beispielen und den Figuren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in den Figuren dargestellt und werden in der nachstehenden Beschreibung näher erläutert. Es zeigen:
- Fig. 1: das Prinzip der Erfassung von abgelagerten Molekülen mit einem Schwingquarz;
- Fig. 2: die Anlagerung von Erythrozyten auf einer schwingungsfähigen ersten Oberfläche;
- Fig. 3a: die Separierung von Antikörpern aus einem Serum;
- Fig. 3b: der Nachweis von Erythrozyten, die Antikörper aus dem Serum gebunden haben; und
- Fig. 4: eine Prinzipdarstellung einer Vorrichtung zum automatischen Durchführen des neuen Verfahrens.

Anhand des nachstehend beschriebenen Beispiels wird die Anwendung des Verfahrens gezeigt.

Auf eine Goldelektrode eines Schwingquarzes (Fa. Vectron International, AT-Schnitt, Durchmesser 8,5 mm, 10 MHz) wird 50 ul Protein-A Lösung (2 mg/ml; Sigma-Aldrich) aufpipettiert. Auf dieser Protein-A Schicht werden anti-A Antikörper BS-66 (Biotest AG) immobilisiert, wobei eine funktionalisierte, gerichtete Oberfläche entsteht, da Protein-A an das Fc-Fragment des BS-66 bindet. Werden bspw. Erythrozyten über den Quarz geführt, so gehen bei Übereinstimmung die Antigene auf der Erythrozytenoberfläche mit den BS-66 eine Bindung ein und werden an die schwingungsfähige Oberfläche gebunden, dort also immobilisiert.

Die Ankopplung der Erythrozyten an die schwingungsfähige Oberfläche kann auch über brückenbildende Moleküle wie z.B. Lektine oder sonstige Makromoleküle, insbesondere auch über Antikörper mit breiter Spezifität außerhalb der gängigen Blutgruppensysteme erfolgen.

Statt der Erythrozyten könne auch andere Trägerpartikel wie z.B. Mikrospheren an die Goldelektrode gebunden werden.

Statt eines Schwingquarzes kann auch ein Balkenschwinger, also ein Cantilever verwendet werden, wie er bspw. beschrieben ist bei Lange et al., Anal. Chem. (2002), 74(13) 3084.

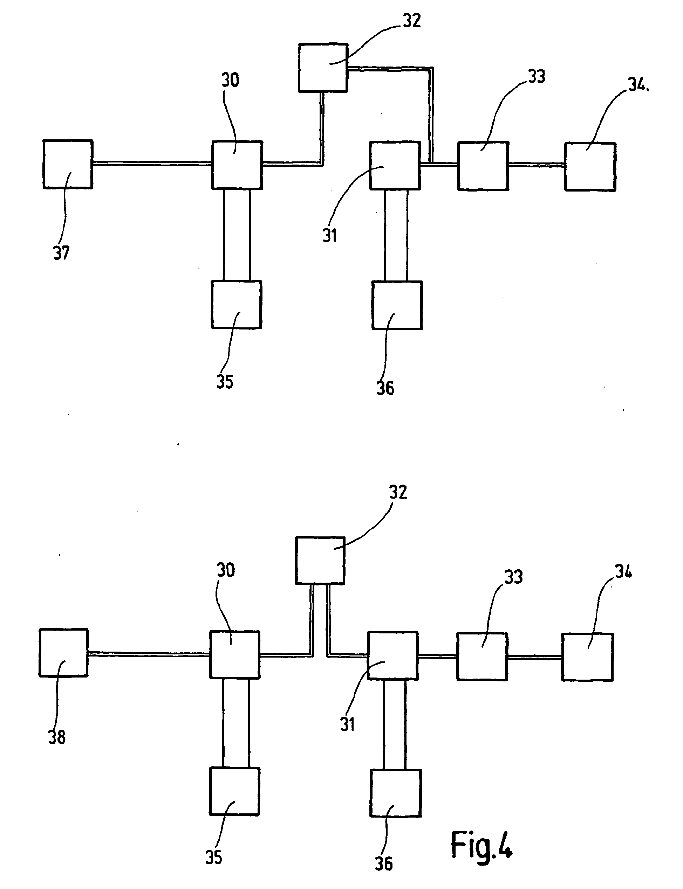

Fig. 4 zeigt einen prinzipiellen Aufbau der neuen Vorrichtung, in der in Zelle 1 ein wie oben beschriebener Schwingquarz eingesetzt wird. Die beiden Zellen 1 und 2, die auch als Messzellen bezeichnet werden, obwohl zur in Zelle 2 eine tatsächliche Messung erfolgt, Zelle 1 dient der Separierung, sind über Ventile und Schläuche miteinander sowie mit Vorratsgefäßen, Probenzufuhr sowie Pumpen verbunden. Eine nur zur Separierung verwendete Vorrichtung würde folglich mit Zelle 1 und entsprechenden Ventilen und Schläuchen etc. auskommen.

In Ventilstellung 1 wird bei einem Antikörpersuchtest zunächst die erste Blutprobe in die Zelle 1 geleitet, so dass Erythrozyten an die Oberfläche binden können. Nach einem Waschschritt wird die zweite Blutprobe in die Zelle 1 geleitet, so dass an die immobilisierten Erythrozyten Antikörper, also Analyten aus der zweiten Probe binden können.

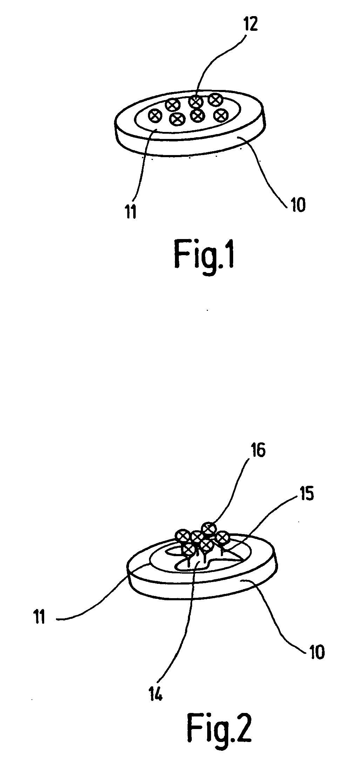

Nach einem weiteren Waschschritt werden die Ventile in Stellung 2 gebracht und durch Erhöhung der Schwingungsamplitude der Oberfläche in Zelle 1 die Erythrozyten mit ggf. daran gebundenen Analyten von den BS-66 abgelöst. Dann wird ein Trägermedium durch Zelle 1 geleitet, das die Trägerpartikel, in diesem Fall also die Erythrozyten in die zweite Zelle transportiert, wo mit Hilfe eines Chemo- oder Biosensors überprüft wird, ob an den Erythrozyten Antikörper gebunden sind. Auch in Zelle 2 kann ein Schwingquarz verwendet werden, der jetzt jedoch bspw. Anti-Humanserum trägt, wie es nachstehend unter Bezug auf die Fig. 1 bis 3 jetzt ausführlicher geschildert wird.

In Fig. 4 oben ist die Ventilstellung 1 gezeigt, in der mit 30 die Zelle 1 und mit 31 die Zelle 2 bezeichnet sind. Zwischen Zelle 1 30 und Zelle 2 31 ist ein Ventil 32 vorgesehen, das Zelle 1 entweder in Ventilstellung 1 mit einer bei 33 angedeuteten Pumpe mit justierbarer Fileßgeschwindigkeit verbindet, die Material aus der Zelle 1 30 in einen Abfallbehälter 34 transportiert.

In der unten in Fig. 4 gezeigten Ventilstellung 2 verbindet das Ventil 32 die Zelle 1 30 mit der Zelle 2 31, so dass Material aus der Zelle 1 30 in die Zelle 2 31 und von dort über die Pumpe 33 in den Abfallbehälter 34 gelangt.

Die Zelle 1 wird über eine bei 35 angedeutete Elektronik angesteuert, die z.B. einen Funktionsgenerator zur Erzeugung einer Sinusspannung mit veränderbarer Amplitude aufweist. Die Zelle 2 31 ist mit einer bei 36 angedeuteten elektronischen Schaltung für die Auswertung der Frequenzveränderung verbunden.

In der Ventilstellung 1 gelangt eine bei 37 angedeutete Probe in die Zelle 1 30, so dass bspw. Erythrozyten an die Oberfläche des dort befindlichen Schwingquarzes binden können. Nach einem Waschschritt wird die zweite Blutprobe in die Zelle 1 30 geleitet, so dass Analyt aus dieser Probe gebunden wird.

In Ventilstellung 2 wird Puffer 38 in die Zelle 1 30 geleitet, um die durch Erhöhung der Schwingungsamplitude der Oberfläche in Zelle 1 30 abgelösten Erythrozyten mit daran ggf. gebundenem Antikörper in Zelle 2 zu leiten.

Schwingquarze sind dafür bekannt, dass sie bei externer Anregung nahe ihrer Resonanzfrequenz nur in einem sehr schmalen Frequenzband eine Amplitudenerhöhung aufweisen. Sie sind daher vorzüglich dafür geeignet, ein schwingendes System, wie z.B. Uhren, exakt auf die Resonanzfrequenz des Quarzes zu justieren. Die Resonanzfrequenz ist nun abhängig von der Geometrie des Quarzes und den Materialeigenschaften (mechanische, piezoelektrische etc.). Zum Beispiel wird bei den sogenannten Dickenscherschwingern, die aufgrund ihrer transversalen Schwingbewegung auch in Flüssigkeitsphase eingesetzt werden können, bei konstanten Materialeigenschaften die Resonanzfrequenz durch die Dicke des Quarzes bestimmt. Insbesondere ist die Resonanzfrequenz auch von der Massenbelegung auf einer der planaren Oberflächen abhängig. Diese Situation ist in Fig. 1 gezeigt, wo ein Quarz 10 mit einer Goldelektrode 11 versehen ist, auf der eine Massenbelegung 12 vorhanden ist.

Wird nun ein Dickenscherschwinger mit einer biologischen Schicht versehen, die spezifisch den Analyten binden kann, so wird bei einer Bindung eine Massenänderung an der Quarzoberfläche generiert. Diese Massenänderung führt zu einer Resonanzfrequenzverschiebung, die elektrisch detektiert werden kann.

Zur Bestimmung der Blutgruppe einer Blutprobe wird genau dieser Effekt ausgenützt. Wie in Fig. 2 gezeigt, wird dazu eine Goldoberfläche 11 zunächst mit Protein A 14 beschichtet und darauf ein spezieller Antikörper 15, z.B. Anti-A, immobilisiert. Wird eine Blutprobe über die schwingende Quarzoberfläche geführt, so binden die Erythrozyten 16 bei Übereinstimmung mit dem entsprechenden Antikörper 15 nach dem Schlüssel-Schloss-Prinzip an die Quarzoberfläche und bewirken die gewünschte Massenänderung.

Nach diesem Prinzip konnten bisher ohne Filterung der Blutprobe die Blutgruppen AB0 und der Rhesusfaktor nachgewiesen werden.

Für eine komplette Blutgruppenbestimmung wird jedoch auch die Gegenprobe und der Antikörper-Suchtest (AKS) verlangt. Bei beiden Verfahren ist es erforderlich, anstatt der schweren Erythrozyten die viel leichteren Antikörper nachzuweisen. Das bringt einige Probleme mit sich, wie es weiter unten noch beschrieben wird.

Um nun Antikörper gegen die Antigene, die auf den Erythrozyten 16 sitzen, nachzuweisen, müssen die Antigene und damit die Erythrozyten 16 auf dem Schwingquarz 10 immobilisiert werden. Dies ist prinzipiell kein Problem, da sie wie oben beschrieben durch eine Antikörper-Antigen-Reaktion an den Quarz gekoppelt werden können; siehe Fig. 2.

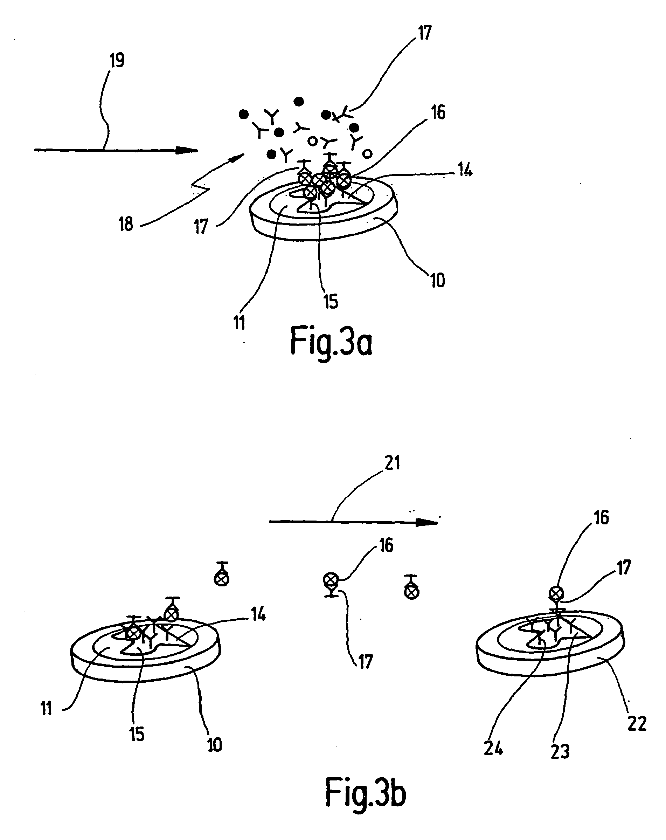

Wird Humanserum 18, das eine Menge an Proteinen und viele verschiedene Antikörper 17 beinhaltet, über den beschichteten Quarz geführt, so reagieren - wie in Fig. 3a gezeigt - die Antikörper 17 mit den Erythrozyten 16 nur dann, wenn auf den immobilisierten Erythrozyten 16 das entsprechende Antigen sitzt. Dies führt bei einer Bindung wiederum zu einer Massenänderung auf der Quarzoberfläche, die theoretisch detektiert werden könnte. Man muss jedoch beachten, dass es auch zu sogenannten unspezifischen Wechselwirkungen kommt, bei denen sich die Proteine und die nicht spezifischen Antikörper des Serums irgendwo an der Quarzoberfläche ablagern. Dadurch und aufgrund der im Gegensatz zur Erythrozytenmasse kleinen Masse der Antikörper 17 ist das Signal nicht aussagekräftig.

Es ist daher vorteilhaft, die spezifischen Antikörper 17 von dem restlichen Serum 18 zu trennen und diese Antikörper 17 an einen Träger mit großer Masse zu koppeln, um ein deutliches Signal zu erhalten. Dies wird jetzt an Hand der Fig. 3 weiter erläutert.

Zuerst werden die Erythrozyten 16 wie beschrieben an die Sensoroberfläche gebunden. Dann wird das Serum 18 über den Quarz 10 geführt, wodurch die spezifischen Bindungen stattfinden können. Anschließend wird die Quarzoberfläche mit einem geeigneten Medium 19 abgespült, wodurch die störenden und unspezifisch reagierenden Proteine und Antikörper entfernt werden; siehe Fig. 3a.

Nun wird die Amplitude des Quarzes durch Erhöhen der angelegten Spannung so gesteigert, dass die Energie ausreicht, um die Bindung zwischen den Erythrozyten 16 und den auf dem Quarz 10 immobilisierten Antikörpern 17 aufzubrechen; siehe Fig. 3b.

Die "abgerissenen" Erythrozyten 16, an deren Oberfläche immer noch die Antikörper 17 aus dem Serum 18 spezifisch gebunden sind, werden mittels einer bei 21 angedeuteten Pufferlösung auf den eigentlichen Transducer transportiert. Dieser weitere Quarz (Transducer) 22 ist mit einer Protein-A-Schicht 23 versehen, auf der Anti-Humanserum 24 immobilisiert ist. Anti-Humanserum 24 hat die Eigenschaft, jeden humanen Antikörper zu binden.

Eine Massenbelegung kann nun beobachtet werden, wenn ein Antikörper 17 aus dem Serum 18 auf dem Erythrozyten gebunden hatte.

Man hat somit erreicht, dass "keine" unspezifischen Wechselwirkungen Einfluss auf die Massenbelegung haben und das Signal nicht durch die leichteren Antikörper 17, sondern den relativ schweren Erythrozyten 16 zustande kommt.

Anstatt der Erythrozyten 16 können auch andere Partikel wie z.B. Latex-Spheres als Antikörper-Träger verwendet werden.

Als zweite Oberfläche kann auch eine aktivierte Goldoberfläche verwendet werden, an der mit Antikörpern beladene Erythrozyten besser binden als unbeladene Erythrozyten. Eine Massebelegung auf der zweiten Oberfläche zeigt dann an, dass Antikörper im ersten Serum vorhanden waren.

Alternativ ist es für den Nachweis, ob in einer ersten Blutprobe Antikörper gegen Erythrozyten aus einer zweiten Blutprobe enthalten sind, auch möglich, zwei schwingungsfähige Oberflächen zu verwenden, die identisch ausgebildet sind, also beide Fängermoleküle für die Erythrozyten enthalten. In einer Vereinfachung ist es sogar möglich, wenn die erste und die zweite schwingungsfähige Oberfläche identisch sind, mit anderen Worten also mit einer einzigen schwingungsfähigen Oberfläche gearbeitet wird.

Dabei wird die Tatsache ausgenutzt, dass mit Antikörpern beladene Erythrozyten an mit Fängermolekülen beschichtete Oberflächen gar nicht oder zumindest mit deutlich geringerer Wahrscheinlichkeit binden, als unbeladene Erythrozyten. Letzteres liegt daran, dass die Antikörper aus dem Serum die Antigene aus den Erythrozyten besetzen oder abdecken, mit denen die Erythrozyten auch an die Fängermoleküle binden.

Bei allen Nachweismethoden wird darüber hinaus noch die Tatsache ausgenutzt, dass die Erythrozyten aus dem zweiten Serum nur die blutgruppenspezifischen Antikörper aus dem ersten Serum binden, auf diese Weise ist für die erforderliche Selektivität gesorgt.

## Patentansprüche

1. Verfahren zum Separieren von zumindest einem Analyten aus einer komplexen, vorzugsweise biologischen Matrix unter Verwendung zumindest einer ersten schwingungsfähigen Oberfläche, mit den Schritten:
a) an der ersten Oberfläche werden Trägerpartikel immobilisiert, die zumindest eine Bindungsstelle für den Analyten aufweisen;
b) die Matrix wird mit der ersten Oberfläche in Kontakt gebracht, so dass ggf. in der Matrix enthaltener Analyt an die Trägerpartikel bindet;
c) unspezifisch gebundene Bestandteile der Matrix werden von der ersten Oberfläche entfernt, vorzugsweise abgewaschen oder abgespült;
d) die erste Oberfläche wird in Schwingung versetzt und ihre Schwingungsamplitude erhöht, bis sich die Trägerpartikel von der Oberfläche ablösen; und
e) die Trägerpartikel mit ggf. daran gebundenem Analyten werden in einem Trägermedium aufgenommen.

2. Verfahren nach Anspruch 1, **dadurch gekennzeichnet, dass** die schwingungsfähige erste Oberfläche an einem Schwingquarz, einem Dickenscherschwinger, einer Quarzmikrowaage oder einem Balkenschwinger ausgebildet ist.

3. Verfahren nach Anspruch 2, **dadurch gekennzeichnet, dass** die schwingungsfähige erste Oberfläche mit einer Elektrode, vorzugsweise einer Goldelektrode versehen ist.

4. Verfahren nach Anspruch 2 oder 3, **dadurch gekennzeichnet, dass** die schwingungsfähige erste Oberfläche bzw. die Elektrode funktionalisiert, vorzugsweise mit einer Beschichtung aus organischem Material versehen ist, insbesondere eine Protein A, Antikörper- oder Lektin-Schicht trägt.

5. Verfahren nach Anspruch 4, **dadurch gekennzeichnet, dass** an der Beschichtung Fängermoleküle, insbesondere Antikörper, Lektine, Biotin oder Avidin gebunden sind, die eine Bindungsstelle für die Trägerpartikel aufweisen.

6. Verfahren nach einem der Ansprüche 1 bis 5, **dadurch gekennzeichnet, dass** die Trägerpartikel über darauf befindliche Liganden oder Antigene an der ersten Oberfläche immobilisiert sind.

7. Verfahren nach einem der Ansprüche 1 bis 6, **dadurch gekennzeichnet, dass** die Trägerpartikel ausgewählt sind aus der Gruppe: Latexpartikel, biologische Makromoleküle, biologische Zellen, Mikroorganismen, Bakterien, Blutzellen, insbesondere Erythrozyten, Partikel aus Zellulose, Polysacchariden, abgetötete Mikroorganismen und Zellen, magnetische Beads.

8. Verfahren nach einem der Ansprüche 1 bis 7, **dadurch gekennzeichnet, dass** jedes Trägerpartikel eine größere, vorzugsweise eine erheblich größere Masse hat als der Analyt.

9. Verfahren nach einem der Ansprüche 1 bis 8, **dadurch gekennzeichnet, dass** der Analyt ausgewählt ist aus der Gruppe: Biomoleküle, insbesondere Antigene oder Antikörper, vorzugsweise Antikörper gegen eine Blutgruppe, gegen Viren, gegen Mikroorganismen oder sonstige Mikropartikel.

10. Verfahren nach einem der Ansprüche 1 bis 9, **dadurch gekennzeichnet, dass** die Matrix ausgewählt ist aus der Gruppe: Körperflüssigkeiten, insbesondere Vollblut, Blutserum, Blutplasma, Urin, Speichel, Tränenflüssigkeit, Liquor cerebrospinalis, Lymphe, Sputum; Zellextrakte; Zellkulturüberstände; wässrige Proben.

11. Verfahren zum Nachweis eines Analyten in einer komplexen, vorzugsweise biologischen Matrix, mit den Schritten:
a) der Analyt wird in einem Verfahren nach einem der Ansprüche 1 bis 10 von der Matrix separiert, und
b) es wird überprüft, ob an die von der ersten Oberfläche abgelösten Trägerpartikel ein Analyt gebunden hat.

12. Verfahren nach Anspruch 11, **dadurch gekennzeichnet, dass** ein gebundener Analyt zu einer messtechnisch erfassbaren Veränderung der biologischen, chemischen oder physikalischen Eigenschaften der Trägerpartikel führt.

13. Verfahren nach Anspruch 11 oder 12, **dadurch gekennzeichnet, dass** die Trägerpartikel über daran gebundenen Analyten an die erste schwingungsfähige Oberfläche oder an einen Chemo- oder Biosensor gebunden werden.

14. Verfahren nach Anspruch 13, **dadurch gekennzeichnet, dass** der Chemo- oder Biosensor als Schwingquarz, Dickenscherschwinger, Quarzmikrowaage oder Balkenschwinger mit einer schwingungsfähigen zweiten Oberfläche ausgebildet ist, die vorzugsweise der ersten schwingungsfähigen Oberfläche entspricht.

15. Verfahren nach Anspruch 14, **dadurch gekennzeichnet, dass** die schwingungsfähige zweite Oberfläche mit einer Elektrode, vorzugsweise einer Goldelektrode, weiter vorzugsweise einer aktivierten Goldoberfläche versehen ist.

16. Verfahren nach Anspruch 14 oder 15, **dadurch gekennzeichnet, dass** die schwingungsfähige zweite Oberfläche bzw. die Elektrode funktionalisiert, vorzugsweise mit einer Beschichtung aus organischem Material versehen ist, insbesondere eine Protein A, Antikörper- oder Lektin-Schicht trägt.

17. Verfahren nach Anspruch 16, **dadurch gekennzeichnet, dass** an der Beschichtung Fängermoleküle, insbesondere Antikörper, Lektine, Biotin oder Avidin gebunden sind, die eine Bindungsstelle für den Analyten aufweisen.

18. Verfahren zum Nachweis von in einer biologischen Probe, vorzugsweise einer Blutprobe enthaltenen Antikörpern/Rezeptoren gegen Antigene/Liganden, unter Verwendung einer ersten schwingungsfähigen Oberfläche, an der Fängermoleküle für Trägerpartikel gebunden sind, auf denen sich die Antigene/Liganden befinden, mit den Schritten:
a) die Trägerpartikel werden mit der ersten Oberfläche in Kontakt gebracht, so dass sie an den Fängermolekülen der ersten Oberfläche immobilisiert werden;
b) die Probe wird mit der Oberfläche in Kontakt gebracht, so dass die Antikörper/Rezeptoren an die Antigene/Liganden auf den Trägerpartikeln binden;
c) unspezifisch gebundene Bestandteile der Probe werden von der Oberfläche entfernt, vorzugsweise abgewaschen oder abgespült;
d) die Oberfläche wird in Schwingung versetzt und ihre Schwingungsamplitude erhöht, bis sich die Trägerpartikel von den Fängermolekülen ablösen;
e) die Trägerpartikel werden mit einer zweiten schwingungsfähigen Oberfläche in Kontakt gebracht, an der Fängermoleküle für die Antikörper/Rezeptoren gebunden sind, wobei vorzugsweise die zweite schwingungsfähige Oberfläche die erste schwingungsfähige Oberfläche ist oder der ersten schwingungsfähigen Oberfläche entspricht bzw. eine aktivierte Goldoberfläche ist; und
f) die zweite schwingungsfähige Oberfläche wird in Schwingung versetzt und ihre Massenbelegung ermittelt.

19. Verfahren zum Nachweis von in einer ersten Blutprobe enthaltenen Antikörpern gegen Erythrozyten in einer zweiten Blutprobe, unter Verwendung einer ersten schwingungsfähigen Oberfläche, an der Fängermoleküle für die Erythrozyten der zweiten Blutprobe gebunden sind, mit den Schritten:
a) die zweite Blutprobe wird mit der Oberfläche in Kontakt gebracht, so dass die Erythrozyten an der Oberfläche immobilisiert werden;
b) unspezifisch gebundene Bestandteile der zweiten Blutprobe werden von der Oberfläche entfernt, vorzugsweise abgewaschen;
c) die erste Blutprobe wird mit der Oberfläche in Kontakt gebracht, so dass die Antikörper an die Erythrozyten binden;
d) unspezifisch gebundene Bestandteile der ersten Blutprobe werden von der Oberfläche entfernt, vorzugsweise abgewaschen oder abgespült;
e) die Oberfläche wird in Schwingung versetzt und ihre Schwingungsamplitude erhöht, bis sich die Erythrozyten von den Fängermolekülen ablösen;
f) die Erythrozyten mit ggf. daran gebundenen Antikörpern werden in einem Trägermedium aufgenommen;
g) das Trägermedium wird mit einer zweiten schwingungsfähigen Oberfläche in Kontakt gebracht, an der Fängermoleküle für die Antikörper gebunden sind, wobei vorzugsweise die zweite schwingungsfähige Oberfläche die erste schwingungsfähige Oberfläche ist oder der ersten schwingungsfähigen Oberfläche entspricht bzw. eine aktivierte Goldoberfläche ist; und
h) die zweite schwingungsfähige Oberfläche wird in Schwingung versetzt und ihre Massenbelegung ermittelt.

20. Verfahren nach Anspruch 19 oder 20 sowie dem Verfahren nach einem oder mehreren der Ansprüche 2 bis 17.

21. Vorrichtung zum Nachweis eines Analyten in einer komplexen, vorzugsweise biologischen Matrix, mit
einer ersten eine schwingungsfähige erste Oberfläche enthaltenden Messzelle, in der ein Analyt aus der Matrix spezifisch an auf einer schwingungsfähigen ersten Oberfläche immobilisierte Trägerpartikel gebunden wird, und Trägerpartikel mit ggf. daran gebundenem Analyten von der Matrix separiert werden;
einer zweiten Messzelle, in der messtechnisch bestimmt wird, ob die Trägerpartikel Analyt gebunden haben; und
einer Einrichtung, um die Trägerpartikel aus der ersten in die zweite Messzelle zu überführen.

22. Vorrichtung nach Anspruch 21, die dazu ausgelegt ist, ein Verfahren nach einem der Ansprüche 1 bis 20 durchzuführen.

23. Vorrichtung nach Anspruch 21 oder 22, **dadurch gekennzeichnet, dass** die Einrichtung Ventile und Schläuche umfasst, über die die einzelnen Flüssigkeiten zu und von den Messzellen geleitet werden.

## Claims

1. Method for separating at least one analyte from a complex, preferably biological matrix using at least one first oscillatory surface, comprising the following steps:
a) carrier particles are immobilized on the first surface, which carrier particles comprise at least one binding site for the analyte;
b) the matrix is brought into contact with the first surface such that the analyte which is possibly contained in the matrix binds to the carrier particles;
c) non-specifically bound components of the matrix are removed, preferably washed or rinsed off, from the first surface;
d) the first surface is made to oscillate and the oscillation amplitude thereof is increased until the carrier particles detach from the surface; and
e) the carrier particles comprising an analyte that is possibly bound thereto are received in a carrier medium.

2. The method of claim 1, **characterized in that** the oscillatory first surface is formed on an oscillator crystal, a thickness shear mode oscillator, a quartz crystal microbalance or a beam oscillator.

3. The method of claim 2, **characterized in that** the oscillatory first surface is provided with an electrode, preferably a gold electrode.

4. The method of claim 2 or 3, **characterized in that** the oscillatory first surface or the electrode, respectively, is functionalized, preferably provided with a coating of organic material, in particular carries a protein A layer, an antibody layer or a lectin layer.

5. The method of claim 4, **characterized in that** capture molecules, in particular antibodies, lectins, biotin or avidin are bound to the coating, which capture molecules comprise a binding site for the carrier particles.

6. The method of one of claims 1 to 5, **characterized in that** the carrier particles are immobilized on the first surface via ligands or antigens present thereon.

7. The method of one of the claims 1 to 6, **characterized in that** the carrier particles are selected from the group: latex particles, biological macromolecules, biological cells, microorganisms, bacteria, blood cells, in particular erythrocytes, particles from cellulose, polysaccharides, deadened microorganisms and cells, magnetic beads.

8. The method of one of claims 1 to 7, **characterized in that** each carrier particle has a larger, preferably a considerably larger mass than the analyte.

9. The method of one of claims 1 to 8, **characterized in that** the analyte is selected from the group: biomolecules, in particular antigens or antibodies, preferably antibodies against a blood group, against viruses, against microorganisms or against other microparticles.

10. The method of one of claims 1 to 9, **characterized in that** the matrix is selected from the group: body fluids, in particular whole blood, blood serum, blood plasma, urine, saliva, lacrimal fluid, liquor cerebrospinalis, lymph, sputum; cell extracts; supernatants of cell cultures; aqueous samples.

11. Method for detecting an analyte in a complex, preferably biological matrix, comprising the steps of:
a) separating the analyte in a method according to one of claims 1 to 10, and
b) determining whether an analyte has bound to the carrier particles detached from the first surface.

12. The method of claim 11, **characterized in that** a bound analyte results in a metrologically detectable change in the biological, chemical or physical characteristics of the carrier particles.

13. The method of claim 11 or 12, **characterized in that** the carrier particles are bound to the first oscillatory surface or to a chemo- or biosensor by means of the analytes bound thereto.

14. The method of claim 13, **characterized in that** the chemo- or biosensor is formed as an oscillator crystal, a thickness shear mode oscillator, a quartz crystal microbalance or a beam oscillator, having an oscillatory second surface, which preferably corresponds to the first oscillatory surface.

15. The method of claim 14, **characterized in that** the oscillatory second surface is provided with an electrode, preferably a gold electrode, and more preferably with an activated gold electrode.

16. The method of claim 14 or 15, **characterized in that** the oscillatory second surface or the electrode, respectively, is functionalized, is preferably provided with a coating of organic material, in particular carries a protein A layer, antibody layer or lectin layer.

17. The method of claim 16, **characterized in that** capture molecules, in particular antibodies, lectins, biotin or avidin, are bound to the coating, which capture molecules comprise a binding side for the analyte.

18. Method for detecting antibodies/receptors against antigens/ligands in a biological sample, preferably in a blood sample, using a first oscillatory surface onto which capture molecules for carrier particles are bound, on which the antigens/ligands are present, comprising the steps of:
a) the carrier particles are brought in contact with the first surface such that they are immobilized on the capture molecules of the first surface;
b) the sample is brought into contact with the surface such that the antibodies/receptors bind to the antigens/ligands on the carrier particles;
c) non-specifically bound components of the sample are removed from the surface, preferably washed or rinsed off;
d) the surface is made to oscillate and the oscillation amplitude thereof is increased until the carrier particles detach from the capture molecules;
e) the carrier particles are brought into contact with a second oscillatory surface, onto which capture molecules for the antibodies/receptors are bound, wherein preferably the second oscillatory surface is the first oscillatory surface or corresponds to the first oscillatory surface or is an activated gold surface, respectively; and
f) the second oscillatory surface is made to oscillate and its mass per unit area is determined.

19. Method for detecting antibodies present in a first blood sample and directed against erythrocytes in a second blood sample, using a first oscillatory surface, onto which capture molecules for the erythrocytes of the second blood sample are bound, comprising the steps of:
a) the second blood sample is brought into contact with the surface such that the erythrocytes are immobilized on the surface;
b) non-specifically bound components of the second blood sample are removed from the surface, preferably washed off;
c) the first blood sample is brought into contact with the surface such that the antibodies bind to the erythrocytes;
d) non-specifically bound components of the first blood sample are removed from the surface, preferably washed or rinsed off;
e) the surface is made to oscillate and the oscillation amplitude thereof is increased until the erythrocytes detach from the capture molecules;
f) the erythrocytes comprising antibodies possibly bound thereto are received in a carrier medium;
g) the carrier medium is brought into contact with a second oscillatory surface onto which the capture molecules for the antibodies are bound, wherein preferably the second oscillatory surface is the first oscillatory surface or corresponds to the first oscillatory surface or is an activated gold surface, respectively; and
h) the second oscillatory surface is made to oscillate and the mass per unit area is determined.

20. The method according to claim 19 or 20 and according to a method of one or several of the claims 2 to 17.

21. Device for detecting an analyte in a complex, preferably biological matrix, having
a first measuring cell containing an oscillatory first surface, in which measuring cell an analyte from the matrix is specifically bound to carrier particles immobilized on an oscillatory first surface, and in which carrier particles comprising analytes possibly bound thereto are separated from the matrix;
a second measuring cell, in which it is metrologically determined, whether the analyte has bound to the carrier particles; and
an equipment to convey carrier particles from the first into the second measuring cell.

22. The device according to claim 21, which is designed to perform a method according to one of claims 1 to 20.

23. The device of claim 21 or 22, **characterized in that** the equipment comprises valves and tubes, by means of which the specific fluids are lead to and from the measuring cells.

## Revendications

1. Procédé en vue de la séparation d'au moins un analyte fait d'une matrice complexe, de préférence, biologique, par utilisation d'au moins une première surface capable d'effectuer des oscillations, comportant les étapes suivantes :
a) des particules de support, qui présentent au moins un point de liaison pour l'analyte, sont immobilisées sur la première surface ;
b) la matrice est mise en contact avec la première surface, de manière à lier, le cas échéant, l'analyte contenu dans la matrice aux particules de support ;
c) les constituants de la matrice liés d'une manière non spécifique sont éliminés, de préférence, sont enlevés, par lavage ou par rinçage, de la première surface ;
d) la première surface est mise en oscillation et son amplitude d'oscillation est augmentée jusqu'à ce que les particules de support se détachent de la surface ; et
e) les particules de support avec, le cas échéant, l'analyte qui y est lié, sont recueillies dans un milieu vecteur.

2. Procédé selon la revendication 1, **caractérisé en ce que** la première surface capable d'effectuer des oscillations est formée d'un cristal oscillateur, d'un oscillateur de cisaillement d'épaisseur, d'une micro balance en quartz ou d'un oscillateur à barres.

3. Procédé selon la revendication 2, **caractérisé en ce que** la première surface capable d'effectuer des oscillations est pourvue d'une électrode, de préférence, d'une électrode en or.

4. Procédé selon la revendication 2 ou 3, **caractérisé en ce que** la première surface capable d'effectuer des oscillations ou l'électrode est fonctionnalisée, est pourvue, de préférence, d'un revêtement en matériau organique, en particulier, porte une protéine A, une couche d'anticorps ou de lectine.

5. Procédé selon la revendication 4, **caractérisé en ce que** sont liés au revêtement des molécules de capture, en particulier des anticorps, de la lectine, de la biotine ou de l'avidine, qui présentent un point de liaison pour les particules de support.

6. Procédé selon l'une quelconque des revendications 1 à 5, **caractérisé en ce que** les particules de support sont immobilisées sur la première surface par l'intermédiaire des ligands ou des antigènes qui s'y trouvent.

7. Procédé selon l'une quelconque des revendications 1 à 6, **caractérisé en ce que** les particules de support sont sélectionnées parmi le groupe constitué de particules de latex, macromolécules biologiques, cellules biologiques, microorganismes, bactéries, cellules sanguines, en particulier, d'érythrocytes, de particules de cellulose, polysaccharides, microorganismes et cellules tuées, perles magnétiques.

8. Procédé selon l'une quelconque des revendications 1 à 7, **caractérisé en ce que** chaque particule de support a une masse plus grande, de préférence, considérablement plus grande que celle de l'analyte.

9. Procédé selon l'une quelconque des revendications 1 à 8, **caractérisé en ce que** l'analyte est sélectionné parmi le groupe constitué de biomolécules, en particulier, d'antigènes ou d'anticorps, de préférence, d'anticorps contre un groupe sanguin, contre des virus, contre des microorganismes ou d'autres microparticules quelconques.

10. Procédé selon l'une quelconque des revendications 1 à 9, **caractérisé en ce que** la matrice est sélectionnée parmi le groupe constitué de liquides corporels, en particulier de sang entier, de sérum sanguin, de plasma sanguin, d'urine, de salive, de liquide lacrymal, de Liquor cerebrospinalis, de lymphe, de crachats, d'extraits cellulaires, de surnageants de cultures cellulaires, d'échantillons aqueux.

11. Procédé en vue de la détection d'un analyte dans une matrice complexe,, de préférence, biologique, grâce aux étapes :
a) l'analyte est séparé de la matrice dans un procédé selon l'une quelconque des revendications 1 à 10, et
b) on contrôle si un analyte s'est lié aux particules de support détachées de la première surface.

12. Procédé selon la revendication 11, **caractérisé en ce qu'**un analyte lié conduit à une modification, détectable par une technique de mesure, des propriétés biologiques, chimiques ou physiques des particules de support.

13. Procédé selon la revendication 11 ou 12, **caractérisé en ce que** les particules de support sont liées à la première surface capable d'effectuer des oscillations ou à un chimio-détecteur ou bio-détecteur à l'aide de l'analyte qui y est lié.

14. Procédé selon la revendication 13, **caractérisé en ce que** le chimio-détecteur ou le bio-détecteur est réalisé sous la forme d'un cristal oscillateur, d'un oscillateur de cisaillement d'épaisseur, d'une micro balance en quartz ou d'un oscillateur à barres ayant une seconde surface capable d'effectuer des oscillations, qui correspond, de préférence, à la première surface capable d'effectuer des oscillations.

15. Procédé selon la revendication 14, **caractérisé en ce que** la seconde surface capable d'effectuer des oscillations est pourvue d'une électrode, de préférence, d'une électrode en or, en outre, de préférence, d'une surface d'or activée.

16. Procédé selon la revendication 14 ou 15, **caractérisé en ce que** la seconde surface capable d'effectuer des oscillations ou l'électrode est fonctionnalisée, de préférence, est pourvue d'un revêtement en matériau organique, en particulier porte une protéine A, une couche d'anticorps ou de lectine.

17. Procédé selon la revendication 16, **caractérisé en ce que** sont liés au revêtement des molécules de capture, en particulier des anticorps, de la lectine, de la biotine ou de l'avidine, qui présentent un point de liaison pour l'analyte.

18. Procédé en vue de la détection des anticorps/des récepteurs contre les antigènes/les ligands, contenus dans un échantillon biologique, de préférence, un échantillon sanguin, avec utilisation d'une première surface capable d'effectuer des oscillations, à laquelle des molécules de capture pour les particules de support sont liées, sur lesquelles les antigènes/les ligands se trouvent, grâce aux étapes :
a) les particules de support sont mises en contact avec la première surface, de façon à les immobiliser sur les molécules de capture de la première surface ;
b) l'échantillon est mis en contact avec la seconde surface, de façon à lier les anticorps/les récepteurs aux antigènes/aux ligands sur les particules de support ;
c) les constituants de l'échantillon non liés d'une manière non spécifique sont éliminés, de préférence, sont enlevés, par lavage ou par rinçage, de la surface ;
d) la surface est mise en oscillation et son amplitude d'oscillation est augmentée jusqu'à ce que les particules de support se détachent des molécules de capture ;
e) les particules de support sont mises en contact avec une seconde surface capable d'effectuer des oscillations, à laquelle les molécules de capture pour les antigènes/les récepteurs sont liées, la seconde surface capable d'effectuer des oscillations étant, de préférence, la première surface capable d'effectuer des oscillations ou correspond à la première surface capable d'effectuer des oscillations ou est une surface d'or activée ; et
f) la seconde surface capable d'effectuer des oscillations est mise en oscillation et sa charge massique est déterminée.

19. Procédé en vue de la détection des anticorps contre les érythrocytes contenus dans un premier échantillon sanguin dans un second échantillon sanguin, avec utilisation d'une première surface capable d'effectuer des oscillations, à laquelle les molécules de capture pour les érythrocytes du second échantillon sanguin sont liées, grâce aux étapes :
a) le second échantillon sanguin est mis en contact avec la surface, de façon à immobiliser les érythrocytes sur la surface ;
b) les constituants non liés d'une manière spécifique du second échantillon sanguin sont éliminés, de préférence, enlevés par lavage de la surface ;
c) le premier échantillon sanguin est mis en contact avec la surface, de façon à lier les anticorps aux érythrocytes ;
d) les constituants de l'échantillon non liés d'une manière non spécifique du premier échantillon sanguin sont éliminés, de préférence, sont enlevés, par lavage ou par rinçage, de la surface ;
e) la surface est mise en oscillation et son amplitude d'oscillation est augmentée jusqu'à ce que les érythrocytes se détachent des molécules de capture ;
f) les érythrocytes avec, le cas échéant, les anticorps qui y sont liés, sont recueillis dans un milieu vecteur ;
g) le milieu vecteur est mis en contact avec une seconde surface capable d'effectuer des oscillations, à laquelle les molécules de capture pour les anticorps sont liées, la seconde surface capable d'effectuer des oscillations étant, de préférence, la première surface capable d'effectuer des oscillations ou correspond à la première surface capable d'effectuer des oscillations ou est une surface d'or activée ; et
h) la seconde surface capable d'effectuer des oscillations est mise en oscillation et sa charge massique est déterminée.

20. Procédé selon la revendication 19 ou 20 ainsi que le procédé selon l'une quelconque des revendications 2 à 17.

21. Dispositif en vue de la détection d'un analyte dans une matrice complexe, de préférence, biologique,
avec une première cellule de mesure, contenant une surface capable d'effectuer des oscillations, dans laquelle un analyte provenant de la matrice est lié d'une manière spécifique aux particules de support immobilisées sur une première surface capable d'effectuer des oscillations et les particules de support avec, le cas échéant, l'analyte qui y est lié étant séparées de la matrice ;
avec une seconde cellule de mesure, dans laquelle on détermine par un technique de mesure si les particules de support ont lié l'analyte ; et
un dispositif destiné au transfert des particules de support hors de la première cellule de mesure dans la seconde cellule de mesure.

22. Dispositif selon la revendication 21, qui est réalisé de manière à exécuter un procédé selon l'une quelconque des revendications 1 à 20.

23. Dispositif selon la revendication 21 ou 22, **caractérisé en ce que ce** dispositif comprend des vannes et des tuyaux souples, via lesquels les liquides individuels sont transférés aux et depuis des cellules de mesure.
